# EUROPEAN PATENT APPLICATION

(11) **EP 2 248 510 A1**
(43) Date of publication of application: **10.11.2010**
(21) Application number: 10161393.3
(22) Date of filing: 29.04.2010
(51) Int. Cl.: A61K 8/26, A61K 8/81, A61K 8/86, A61Q 5/06, A61K 8/04

(54) **Thickened aqueous composition**

(30) Priority: 07.05.2009 EP 09290334
(71) Applicant: Rohm and Haas Company, Philadelphia, Pennsylvania 19106-2399 (US)
(72) Inventor: Breton, Marie-Laure, 06410, Biot (FR); Torre, Frederic De La, 06600, Antibes (FR)
(74) Representative: Buckley, Guy Julian

(57) **Abstract**

An aqueous composition having less than 1 wt% surf act ant . The aqueous composition contains a first polymer, a second polymer and a synthetic clay. The first polymer contains (meth)acrylate ester(s) of a polyethylene glycol having 15 to 30 et hyl ene oxide residues and a C₁₀-C₂₂ alkyl group on one end. The second polymer contains C₃-C₆ carboxylic acid monomer(s) and C₂-C₃ hydroxyalkyl (meth)acrylate(s).

## Description

This invention relates to an aqueous composition containing rheology-modifying thickeners.

Rheology modifiers are used in aqueous cleaning products, including for example, shampoo, to increase viscosity at low shear rates while maintaining flow properties of the product at higher shear rates. A variety of copolymer thickeners made from vinyl monomers have been used for this purpose. For example, U.S. Pat. No. 7,132,468 discloses a composition containing at least 18% surfactant, a lipophilically-modified acrylic copolymer and a clay. However, thickeners providing an optimum viscosity profile at low surfactant levels are greatly needed to provide thickening and suspending properties to a variety of other personal care products. Particularly desired is a large increase in low shear viscosity without substantial increase in high shear viscosity, accompanied by low turbidity.

The problem addressed by the present invention is the need for rheology-modifying polymers providing optimum viscosity profiles in low-surfactant aqueous compositions.

### STATEMENT OF INVENTION

The present invention provides an aqueous composition having less than 1 wt% surfactant comprising: (a) from 0.5-4 wt% of a first polymer comprising polymerized residues of: (i) from 5-30 wt% (meth)acrylate ester of a polyethylene glycol having 15 to 30 ethylene oxide residues and a C₁₀-C₂₂ alkyl group on one end; (ii) from 10-50% (meth)acrylic acid; and (iii) from 40-65 wt% C₁-C₈ alkyl (meth)acrylate; (b) from 0.05-1.5 wt% of a synthetic clay having an average particle size from 20-90 nm and a surface area from 120-500 m²/g; and (c) from 0.3-5 wt% of a second polymer comprising polymerized residues of: (i) from 5-35 wt% C₃-C₆ carboxylic acid monomer; (ii) from 5-20 wt% C₂-C₃ hydroxyalkyl (meth)acrylate; (iii) from 20-55 wt% C₁-C₄ alkyl methacrylate; and (iv) from 18-55 wt% C₂-C₄ alkyl acrylate; wherein the aqueous composition has a pH from 5 to 10.

### DETAILED DESCRIPTION

Percentages are weight percentages (wt%) and temperatures are in °C, unless specified otherwise. Viscosities were measured using an AR1000 rheometer from TA Instruments equipped with a 4°, 40 mm cone and operating at a temperature of approximately 20°C. Particle sizes are measures of the largest particle dimension, e.g., the diameter for disc-shaped particles. Values of pH are those measured at approximately 20°C.

As used herein the term "(meth)acrylic" refers to acrylic or methacrylic, and "(meth)acrylate" refers to acrylate or methacrylate. "Acrylic monomers" include acrylic acid (AA), methacrylic acid (MAA), esters of AA and MAA, acrylamide (AM), methacrylamide (MAM), and derivatives of AM and MAM, e.g., octyl acrylamide (OAM). Esters of AA and MAA include, but are not limited to, alkyl, hydroxyalkyl and sulfoalkyl esters, e.g., methyl methacrylate (MMA), ethyl methacrylate (EMA), butyl methacrylate (BMA), hydroxyethyl methacrylate (HEMA), hydroxyethyl acrylate (HEA), isobornyl methacrylate (IBOMA), methyl acrylate (MA), ethyl acrylate (EA), butyl acrylate (BA), and longer chain alkyl (meth)acrylates such as ethylhexyl acrylate (EHA), lauryl methacrylate (LMA), lauryl acrylate (LA), cetyl methacrylate (CEMA), and stearyl methacrylate (SMA). The term "(meth)acrylamide" refers to acrylamide (AM) or methacrylamide (MAM). Derivatives of (meth)acrylamide include, but are not limited to, alkyl- and sulfoalkyl-substituted (meth)acrylamides, e.g., N,N-dimethyl acrylamide, N,N-dipropyl acrylamide, t-butyl acrylamide, N-octyl acrylamide, 2-acrylamido-2-methylpropanesulfonic acid (AMPS), and longer chain alkyl (meth)acrylamides such as N-lauryl methacrylamide, N-stearyl methacrylamide.

The term "vinyl monomers" refers to monomers that contain a carbon-carbon double bond that is connected to a heteroatom such as nitrogen or oxygen. Examples of vinyl monomers include, but are not limited to, vinyl acetate, vinyl formamide, vinyl acetamide, vinyl pyrrolidone, vinyl caprolactam, and long chain vinyl alkanoates such as vinyl neodecanoate, and vinyl stearate.

The term "acrylic polymers" refers to polymers of acrylic monomers, and copolymers comprising at least 50% of acrylic monomers and (meth)acrylamide monomers. Preferably, acrylic polymers have at least 75% of monomer residues derived from (meth)acrylic acid or (meth)acrylate or (meth)acrylamide monomers, more preferably at least 85%, and most preferably at least 95%. Preferably, the remaining monomer units are derived from vinyl monomers, styrene or α-methylstyrene. The term "acrylamide polymers" refers to polymers of (meth)acrylamide monomers, and copolymers comprising at least 50% of (meth)acrylamide monomers. Preferably, acrylamide polymers have at least 70% of monomer residues derived from (meth)acrylamide monomers, and most preferably at least 85%.

For purposes of this invention, alkyl groups are straight or branched chain alkyl groups or aralkyl or alkyl carbocyclic groups, such as alkylphenyl groups. It is understood that the alkyl groups may be either of synthetic or of natural origin and, in the latter case particularly, may contain a range of chain lengths. For example, naturally sourced stearic acid, even of commercially pure quality may contain only about 90% of stearic chains, up to about 7% of palmitic chains and a proportion of other chains and lower quality products may contain substantially less stearic acid. Polyethylene glycols also have a distribution of chain lengths having different numbers of polymerized ethylene oxide residues. It is intended herein that reference to the chain length of natural mixed alkyl groups is to the predominant chain length which is present as more than 50%, preferably in more than 75%, of the chains. In some embodiments of the invention, alkyl groups are straight or branched chain acyclic alkyl groups. Reference to the number of ethylene oxide residues in a polyethylene glycol is to the number of ethylene oxide residues corresponding to Mn of the polyethylene glycol.

A (meth)acrylate ester of a polyethylene glycol having 15 to 30 ethylene oxide (EO) residues and a C₁₀-C₂₂ alkyl group on one end, in the first polymer of this invention, is a (meth)acrylate ester in which the group attached to the (meth)acryloyl group contains one hydroxyl group. Polyethylene glycols contain two hydroxyl groups, one on each end of the chain. The group attached to the (meth)acryloyl group in this invention is "capped" on one end with a C₁₀-C₂₂ alkyl group by formation of a C₁₀-C₂₂ alkyl ether. A general structure for this monomer would be: CH₂=CR¹CO₂(CH₂CH₂O)ₙR², wherein R¹ is hydrogen or methyl, n is 15-30 and R² is a C₁₀-C₂₂ alkyl group, i.e., in the case of a methacryloyl ester, 20 EO residues and a C₁₈ alkyl, a monomer structure would be: CH₂=CHCO₂(CH₂CH₂O)₂₀C₁₈H₃₇. Of course other structures with different numbers of EO units, and possibly, different alkyl chain lengths, may also be present. In some embodiments of the invention, the alkyl group has from 12 to 22 carbon atoms, alternatively from 16 to 22 carbon atoms, alternatively from 18 to 22 carbon atoms. In some embodiments of the invention, the (meth)acryloyl ester is a methacryloyl ester. In some embodiments of the invention, the first polymer contains polymerized residues of at least 10% (meth)acrylate ester of a polyethylene glycol having 15 to 30 EO residues and a C₁₀-C₂₂ alkyl group on one end, alternatively at least 12%, alternatively at least 15%, alternatively at least 18%, alternatively at least 20%, alternatively at least 22%; alternatively no more than 28%, alternatively no more than 25%. In some embodiments, the ester contains at least 17 EO residues, alternatively at least 20; alternatively no more than 28, alternatively no more than 23.

In some embodiments of the invention, the first polymer contains at least 15% polymerized (meth)acrylic acid monomer residues, alternatively at least 18%, alternatively at least 22%. In some embodiments of the invention, the polymer contains no more than 45% (meth)acrylic acid monomer residues, alternatively no more than 40%, alternatively no more than 35%, alternatively no more than 30%, alternatively no more than 27%. The first polymer may contain other C₃-C₆ carboxylic acid monomers, preferably in a total amount no greater than 5%, alternatively no greater than 3%, alternatively no greater than 1%. In some embodiments of the invention, the first polymer contains from 1-10% methacrylic acid residues, alternatively from 2-8%, alternatively from 3-7%. In some embodiments of the invention, the first polymer contains from 10-28% polymerized acrylic acid residues, alternatively from 13-25%, alternatively from 15-23%.

In some embodiments of the invention, the first polymer contains at least 43% polymerized residues of C₁-C₈ alkyl (meth)acrylate(s), alternatively at least 46%, alternatively at least 48%. In some embodiments, the first polymer contains no more than 60% C₁-C₈ alkyl (meth)acrylate, alternatively no more than 55%. In some embodiments of the invention, the C₁-C₈ alkyl (meth)acrylate is a C₁-C₄ alkyl (meth)acrylate, alternatively a C₁-C₄ alkyl acrylate, alternatively methyl acrylate or ethyl acrylate.

In some embodiments of the invention, the first polymer is crosslinked, that is, at least one crosslinker, such as a monomer having two or more ethylenically unsaturated groups, is included with the copolymer components during polymerization. Crosslinkers include, e.g., divinylaromatic compounds, di- and tri-(meth)acrylate esters, di- and tri-allyl ether or ester compounds, allyl (meth)acrylate. Preferred examples of such monomers include divinylbenzene (DVB), trimethylolpropane diallyl ether, tetraallyl pentaerythritol, triallyl pentaerythritol, diallyl pentaerythritol, diallyl phthalate, triallyl cyanurate, Bisphenol A diallyl ether, allyl sucroses, methylene bisacrylamide (MBA), trimethylolpropane triacrylate, allyl methacrylate. In one embodiment, the crosslinker does not have ester functionality. In some embodiments of the invention, the amount of polymerized crosslinker residue in the polymer is from 0.01 % to 1 %, based on the total weight of the monomers. In some embodiments of the invention, the amount of crosslinker residue in the polymer is no more than 0.7%, alternatively no more than 0.5 %, alternatively no more than 0.3 %, alternatively no more than 0.2%. In some embodiments, the amount of crosslinker residue in the polymer is at least 0.03%, alternatively at least 0.05%, alternatively at least 0.07%. In some embodiments of the invention, the crosslinker is diethylenically unsaturated, e.g., trimethylolpropane diallyl ether or DVB.

In some embodiments of the invention, the aqueous composition comprises at least 0.8% of the first polymer, alternatively at least 1 %, alternatively at least 1.2%, alternatively at least 1.3%, alternatively at least 1.4%, alternatively at least 1.5%. In some embodiments, the composition comprises no more than 3% of the first polymer, alternatively no more than 2.7%, alternatively no more than 2.4%, alternatively no more than 2.2%, alternatively no more than 2.1%, alternatively no more than 2%, alternatively no more than 1.9%. In some embodiments, more than one first polymer may be present, with the total amount of first polymers being within the amounts specified above.

In some embodiments of the invention, the second polymer contains at least 10% polymerized C₃-C₆ carboxylic acid monomer residues, alternatively at least 12%, alternatively at least 14%. In some embodiments, the second polymer contains no more than 30% polymerized C₃-C₆ carboxylic acid monomer residues, alternatively no more than 28%, alternatively no more than 26%, alternatively no more than 23%. In some embodiments of the invention, the C₃-C₆ carboxylic acid monomer is a C₃-C₅ carboxylic acid monomer; alternatively it is acrylic acid, methacrylic acid, itaconic acid or crotonic acid; alternatively it is acrylic acid, methacrylic acid or itaconic acid. In some embodiments of the invention, the second polymer contains from 5-35% polymerized residues of methacrylic acid, alternatively from 7-30%, alternatively from 10-25%, alternatively from 10-17%, alternatively from 18-30%. In some embodiments, the second polymer contains from 1-10% polymerized residues of itaconic acid, alternatively from 2-8%, alternatively from 3-7%.

In some embodiments of the invention, the second polymer contains at least 7% polymerized residues of C₂-C₃ hydroxyalkyl (meth)acrylate(s), alternatively at least 8%, alternatively at least 9%. In some embodiments, the second polymer contains no more than 17% polymerized residues of C₂-C₃ hydroxyalkyl (meth)acrylate(s), alternatively no more than 15%, alternatively no more than 14%, alternatively no more than 13%, alternatively no more than 12%. In some embodiments of the invention, the C₂-C₃ hydroxyalkyl (meth)acrylate(s) are hydroxyethyl (meth)acrylate(s), alternatively hydroxyethyl methacrylate.

In some embodiments of the invention, the second polymer contains at least 23% polymerized residues of C₁-C₄ alkyl methacrylate(s), alternatively at least 25%, alternatively at least 30%, alternatively at least 35%, alternatively at least 40%, alternatively at least 42%. In some embodiments, the second polymer contains no more than 52% polymerized residues of C₁-C₄ alkyl methacrylate(s), alternatively no more than 51%, alternatively no more than 50%. In some embodiments of the invention, the second polymer contains from 42-50% polymerized residues of C₁-C₄ alkyl methacrylate(s) and in other embodiments, from 23-32%. In some embodiments of the invention, the C₁-C₄ alkyl methacrylate(s) are methyl methacrylate, ethyl methacrylate or combinations thereof; alternatively methyl methacrylate.

In some embodiments of the invention, the second polymer contains at least 20% polymerized residues of C₂-C₄ alkyl acrylate(s), alternatively at least 22%, alternatively at least 25%, alternatively at least 30%. In some embodiments, the second polymer contains no more than 50% polymerized residues of C₂-C₄ alkyl acrylate(s), alternatively no more than 47%, alternatively no more than 40%, alternatively no more than 35%, alternatively no more than 30%. In some embodiments of the invention, the second polymer contains from 20-30% polymerized residues of C₂-C₄ alkyl acrylate(s) and in other embodiments, from 37-47%. In some embodiments of the invention, the C₂-C₄ alkyl acrylate(s) are ethyl acrylate, butyl acrylate or combinations thereof; alternatively butyl acrylate. Small amounts of methyl acrylate and/or higher alkyl acrylates, e.g., up to 10%, may also be present.

In some embodiments of the invention, the aqueous composition comprises at least 0.4% of the second polymer, alternatively at least 0.5%, alternatively at least 0.8%, alternatively at least 1%, alternatively at least 1.4%, alternatively at least 1.7%. In some embodiments, the composition comprises no more than 4% of the second polymer, alternatively no more than 3.5%, alternatively no more than 3%, alternatively no more than 2.8%, alternatively no more than 2.7%, alternatively no more than 2.6%, alternatively no more than 2.5%. In some embodiments, more than one second polymer may be present, with the total amount of second polymers being within the amounts specified above.

The polymers used in this invention may be prepared by copolymerizing the monomers using well known precipitation, reverse emulsion, gel polymerization processes, and any other suitable processes known in the art, using, for example, a free-radical initiator such as peroxygen compounds or diazo compounds and, optionally, chain transfer agents.

The length of the primary polymer chains is typically such that, if any crosslinks were removed, the molecular weight (M_{w}) would be in the range of about 50,000 to 10,000,000 for the first polymer, alternatively from 100,000 to 5,000,000, alternatively from 200,000 to 2,000,000; and from 25,000 to 250,000 for the second polymer, alternatively from 40,000 to 150,000.

In some embodiments of the invention, the pH of the aqueous composition is at least 6, alternatively at least 6.5, alternatively at least 7. In some embodiments of the invention, the pH is no more than 9, alternatively no more than 8. Neutralization of the carboxylic acid functional groups on the polymers to achieve the desired pH may be done with inorganic bases, e.g., alkali metal or alkaline earth metal hydroxides, e.g., NaOH or KOH; or with amines, e.g., alkanol amine compounds, e.g., C₃-C₁₂ alkanol amine compounds, e.g., 2-amino-2-methyl-1-propanol (AMP), aminomethyl propanediol (AMPD), triethanolamine (TEA), triisopropanolamine (TIPA).

Preferably, the first and second polymers are acrylic polymers. The polymers may be blended into an aqueous system to be thickened together with a suitable addition of acidic or basic material if required. Preferably, the aqueous composition has at least 65% water, alternatively at least 75%, alternatively at least 80%. Preferably, the aqueous composition contains no more than 30% organic solvent, alternatively no more than 20%, alternatively no more than 15%, alternatively no more than 10%. In some embodiments, the aqueous composition is substantially free of organic solvents. Typical organic solvents which could be present in the composition include, e.g., alcohols, including ethanol and isopropanol, alternatively ethanol. In some embodiments, the composition contains at least 1% organic solvent, alternatively at least 5%, alternatively at least 10%. In some embodiments of the invention, the aqueous composition contains no more than 0.7% surfactant, alternatively no more than 0.5%, alternatively no more than 0.3%.

The term "surfactants," as used herein refers to anionic, cationic, zwitterionic and nonionic surfactants. Typically, surfactants contain an alkyl group having at least eight carbon atoms and either: (i) an anionic and/or cationic group; or (ii) a chain containing polymerized alkylene oxides, e.g., ethylene oxide and/or propylene oxide. Examples of anionic surfactants include those having carboxyl, sulfonate, sulfate or phosphate functional groups. Examples of cationic surfactants include those having amine, pyridinium or ammonium functional groups. Examples of zwitterionic surfactants include those having combinations of the anionic and cationic functional groups mentioned above. Examples of nonionic surfactants include those having amide or hydroxyl functional groups and those having polymerized residues of ethylene oxide and/or propylene oxide. For purposes of this invention, polymers having a molecular weight, Mw, of at least 20,000 are not considered to be surfactants, regardless of functionality. Moreover, polymers containing less than 40% of polymerized residues having any of the functional groups described above for surfactants are not considered to be surfactants. Typically, surfactants contain at least one C₈-C₂₂ alkyl or aralkyl group.

In some embodiments of the invention, the aqueous composition contains at least 0.1% synthetic clay(s), alternatively at least 0.2%, alternatively at least 0.3%, alternatively at least 0.4%. In some embodiments, the composition contains no more than 1.2% synthetic clay(s), alternatively no more than 1%, alternatively no more than 0.9%, alternatively no more than 0.8%, alternatively no more than 0.7%, alternatively no more than 0.6%. In some embodiments of the invention, the synthetic clay has an average particle size of at least 22 nm, alternatively at least 25nm, alternatively at least 30 nm, alternatively at least 35 nm. In some embodiments, the average particle size is no more than 85 nm, alternatively no more than 80 nm, alternatively no more than 70 nm, alternatively no more than 60 nm, alternatively no more than 50 nm, alternatively no more than 40 nm, alternatively no more than 30 nm. In some embodiments of the invention, the synthetic clay(s) has a surface area of at least 140 m²/g, alternatively at least 180 m²/g, alternatively at least 250 m²/g, alternatively at least 300 m²/g, alternatively at least 340 m²/g. In some embodiments, the synthetic clay(s) has a surface area of no more than 450 m²/g, alternatively no more than 420 m²/g. In some embodiments of the invention, the synthetic clay comprises particles in the form of discs with a thickness of 0.5-2 nm and a diameter of about the average particle size.

The composition of the present invention optionally may include other ingredients, e.g., salts, co-rheology modifiers (e.g. cellulosics, carrageenan, xanthan, PEG-150 distearate, PEG-150 pentaerythrityl tetrastearate, other associative or non-associative rheology modifiers, polymeric quats (e.g., PQ-7 and PQ-10), dispersants, silicones, soluble or dispersed biocides, vitamins, humectants, enzymes, emollient, fragrance, dyes, thioglycolic acid, UVA and UVB absorbers, infrared absorbers, etc.

Particular uses for the aqueous composition of this invention include hair gel (alcohol-containing and alcohol-free); hair styling spray, spray gel, cream, paste, or gum; sunscreen lotions and sprays, tanning lotions, skin care lotions, skin care lotions containing vitamins, two-part hair dyes, permanent waving formulations, and thickening all types of alcohol or water/alcohol formulations.

### EXAMPLES

Monomer percentages in polymers are calculated on the basis of total monoethylenically unsaturated monomers, excluding crosslinkers and chain transfer agents. Percentages of crosslinkers and chain transfer agents are calculated on the basis of total monoethylenically unsaturated monomers. Accordingly, those polymers including chain transfer agents and crosslinkers will have total percentages greater than 100%, but one can easily normalize the percentages to 100%. Low-shear (0.1-0.5 Pa) and high-shear (100-500 Pa) viscosities were measured at 0.1 Pa and 500 Pa, respectively, unless otherwise indicated, and the viscosities presented in Pa·s

### Comparative Example 1: Aqueous Compositions without the First Polymer

The following compositions were prepared and low-shear and high-shear viscosities were measured and the results presented in Table 1 below.

**Table 1**

| | | | |
|---|---|---|---|
| Water | qs100 | qs100 | qs100 |
| TEA | 0.40 | 0.80 | 0.85 |
| Second polymer #1 | 1.38 | 1.38 | 1.38 |
| Water | 15.00 | 25.00 | 25.00 |
| LAPONITE® XLG | 0.30 | 0.60 | 0.90 |
| NEOLONE™PE | 0.50 | 0.50 | 0.50 |
| | | | |
| Low shear stress viscosity | 2x10⁻³ | 0.01 | 5 |
| High Shear stress viscosity | 4x10⁻³ | 5x10⁻³ | 7x10⁻³ |

| | | | |
|---|---|---|---|
| Notes: "qs100" indicates addition of water to a total composition volume of 100 mL. Second polymer #1 is a copolymer of 13% MAA, 5% itaconic acid, 47% MMA, 25% BA and 10% HEMA, with 0.6% n-dodecylmercaptan (DDM) as a chain transfer agent; 46% solids aqueous dispersion; Mw=120,000. NEOLONE PE is a biocide. TEA is triethanolamine. LAPONITE XLG is a synthetic clay with surface area 370 m²/g, particle size 25 nm. | | | |

The formulations in Table 1 all have unacceptably low viscosity.

### Example 1: Comparison of Different Clays

Turbidity was measured using a HACH RATIO/XR turbidimeter at approximately 20-25°C. The turbidimeter directs light through the test sample in a cylindrical cell. Detectors measure scattered light at 90° to the incident light, forward scattered light and transmitted light. The results are reported in NTU.

**Table 2**

| | | | | | | | |
|---|---|---|---|---|---|---|---|
| Water | qs100 | qs100 | qs100 | qs100 | qs100 | qs100 | qs100 |
| TEA | 1.82 | 1.69 | 1.79 | 1.78 | 1.86 | 1.70 | 1.99 |
| Second polymer #1 | 5.00 | 5.00 | 5.00 | 5.00 | 5.00 | 5.00 | 5.00 |
| Water | 15.00 | 15.00 | 25.00 | 25.00 | 15.00 | 25.00 | 25.00 |
| LAPONITE® XLG | - | 0.30 | 0.50 | 0.90 | - | - | - |
| LAPONITE® OG | - | - | - | - | 0.30 | 0.50 | - |
| LAPONITE® XLS | - | - | - | - | - | - | 0.50 |
| First polymer #1 | 6.00 | 6.00 | 6.00 | 6.00 | 6.00 | 6.00 | 6.00 |
| NEOLONE™PE | 0.50 | 0.50 | 0.50 | 0.50 | 0.50 | 0.50 | 0.50 |
| | | | | | | | |
| Low shear viscosity | 4x10⁴ | 5x10⁵ | 1x10⁶ | 3x10⁶ | 1x10⁶ | 3x10⁶ | 1x10⁶ |
| High shear viscosity | 20 | 10 | 10 | 3 | 10 | 4 | 10 |
| turbidity | 7 | 10.7 | 16.7 | 50.1 | 51.3 | 89.3 | 19.0 |

| | | | | | | | |
|---|---|---|---|---|---|---|---|
| Notes: First polymer #1 is a copolymer of 5% MAA, 19%AA, 51% EA and 25% methacrylate ester of a polyethylene glycol having 20 ethylene oxide residues and a C₁₈ alkyl group on one end, with 0.1% trimethylolpropane diallyl ether and 0.1% DDM; 29% solids dispersion in water; hydrolyzed Mw=400,000. LAPONITE OG is a synthetic clay with surface area 150 m²/g, particle size 83 nm | | | | | | | |

Low shear viscosity was increased by a factor of 10-75, while high shear viscosity was the same or was decreased slightly. Turbidity values greater than 80 are not acceptable in most applications. Values less than 60 are preferred.

### Example 2: Different First Polymers

**Table 3**

| | | | | | | |
|---|---|---|---|---|---|---|
| Water | qs100 | qs100 | qs100 | qs100 | qs100 | qs100 |
| TEA | 1.37 | 1.47 | 1.48 | 1.86 | 1.86 | 1.93 |
| Second polymer | 5.00 | 5.00 | 5.00 | 5.00 | 5.00 | 5.00 |
| #1 | | | | | | |
| Water | 15.00 | 25.00 | 15.00 | 15.00 | 25.00 | 15.00 |
| LAPONITE®XLG | - | 0.70 | - | - | 0.50 | - |
| LAPONITE®OG | - | - | 0.40 | - | - | 0.30 |
| First Polymer #2 | 5.00 | 5.00 | 5.00 | - | - | - |
| First Polymer #3 | - | - | - | 5.00 | 5.00 | 5.00 |
| NEOLONE™PE | 0.5 | 0.5 | 0.5 | 0.5 | 0.5 | 0.5 |
| | | | | | | |
| Low shear stress viscosity | 2000 | 1x10⁶ | 1x10⁵ | 800 | 7x10⁵ | 4x10⁵ |
| High Shear stress viscosity | 0,01 | 0.01 | 0.01 | 50 | 20 | 20 |
| Turbidity | 22.0 | 35.8 | 58.9 | 6.7 | 14.8 | 43.4 |

First polymer #2 is a copolymer of 5% MAA, 19%AA, 57% MA and 19% methacrylate ester of a polyethylene glycol having 25 ethylene oxide residues and a C₂₂ alkyl group on one end; 20% solids dispersion in water; Mw=750,000. First polymer #3 is a copolymer of 37% MAA, 49% EA and 14% methacrylate ester of a polyethylene glycol having 20 ethylene oxide residues and a C₁₈ alkyl group on one end; 29% solids dispersion in water; Mw=750,000.

The influence of addition of the clays on low shear viscosity is also visible with First Polymers #2 and #3 with low shear viscosity increased by a factor 50 to 500 while high shear viscosity remains unchanged.

### Comparative Example 2: Compositions with Alternate Polymers in Place of First Polymer High shear viscosities were measured at 100 Pa shear.

**Table 4**

| | | | | | |
|---|---|---|---|---|---|
| Water | qs100 | qs100 | qs100 | qs100 | qs100 |
| TEA | 2.10 | 2.18 | 2.38 | 2.31 | 2.08 |
| Second polymer #1 | 5.00 | 5.00 | 5.00 | 5.00 | 5.00 |
| Water | 15.00 | 15.00 | 15.00 | 15.00 | 15.00 |
| LAPONITE®XLG | - | 0.70 | - | - | 0.50 |
| LAPONITE®OG | - | - | 0.40 | - | - |
| Comp. first poly. #1 | 6.86 | 6.86 | 6.86 | - | - |
| Comp. first poly. #2 | - | - | - | 6.86 | 6.86 |
| NEOLONE PE | 0.50 | 0.50 | 0.50 | 0.50 | 0.50 |
| Low shear stress viscosity | 5x10⁴ | 2x10⁶ | 4x10⁵ | 9x10⁴ | 1x10⁶ |
| High shear stress viscosity | 0.1 | 1 | 2 | 0.1 | 5 |
| turbidity | 31.5 | 87.1 | 51.9 | 61 | 182.9 |

Comp. first poly. #1 is a copolymer of 36% MAA, 56% EA, 5% EHA and 3% vinyl neodecanoate, with 0.1% trimethylolpropane diallyl ether; 28% solids dispersion in water; Mw=800,000. Comp. first poly. #2 is a crosslinked copolymer of 36% MAA and 64% EA.

Low shear viscosity was increased with the addition of the clays by a factor of 8 to 40, with a simultaneous increase in high shear viscosity by a factor of 10 to 20. No synergy of thickeners was observed, only the additive effects.

### Example 3: Different Grades of Clay

**Table 5**

| | | | | |
|---|---|---|---|---|
| Water | qs100 | qs100 | qs100 | qs100 |
| TEA | 1.82 | 1.70 | 1.80 | 1.81 |
| Second polymer #1 | 5.00 | 5.00 | 5.00 | 5.00 |
| Water | 15.00 | 25.00 | 15.00 | 15.00 |
| LAPONITE OG | - | 0.50 | - | - |
| LAPONITE RD | - | | 0.50 | - |
| LAPONITE SL25 * | - | | - | 2.00 |
| First poly. #1 | 6.00 | 6.00 | 6.00 | 6.00 |
| NEOLONE PE | 0.50 | 0.50 | 0.50 | 0.50 |
| Low shear stress viscosity | 4x10⁴ | 3x10⁶ | 2x10⁶ | 7x10⁵ |
| High shear stress viscosity | 20 | 4 | 10 | 10 |
| turbidity | 7 | 89.3 | 23.4 | 17.8 |

| | | | | |
|---|---|---|---|---|
| * 25% solids dispersion in water; LAPONITE RD is a synthetic clay with surface area 369 m²/g, particle size 45 nm; LAPONITE SL25 is a synthetic clay with surface area 370 m²/g, particle size 45 nm | | | | |

### Example 4: Lower Amounts of LAPONITE OG with Polymer #1

**Table 6**

| | | | |
|---|---|---|---|
| Water | qs100 | qs100 | qs100 |
| TEA | 1.82 | 1.58 | 1.67 |
| Second polymer #1 | 5.00 | 5.00 | 5.00 |
| Water | 15.00 | 15.00 | 25.00 |
| LAPONITE® OG | - | 0.20 | 0.30 |
| First polymer #1 | 6.00 | 5.00 | 5.00 |
| NEOLONE™PE | 0.50 | 0.50 | 0.50 |
| | | | |
| Low shear viscosity | 4x10⁴ | 5x10⁵ | 1x10⁶ |
| High shear viscosity | 20 | 20 | 20 |
| turbidity | 7 | 41.1 | 55.6 |

### Example 5: Different Amounts of Second Polymer #1

**Table 7**

| | | | | |
|---|---|---|---|---|
| Water | qs100 | qs100 | qs100 | qs100 |
| TEA | 1.11 | 1.02 | 1.15 | 1.73 |
| Second polymer #1 | 0 | 0.5 | 1 | 5 |
| Water | 15.00 | 15.00 | 15.00 | 15.00 |
| LAPONITE® XLG | 0.5 | 0.5 | 0.5 | 0.5 |
| First polymer #1 | 6.00 | 6.00 | 6.00 | 6.00 |
| NEOLONE™PE | 0.50 | 0.50 | 0.50 | 0.50 |
| | | | | |
| Low shear viscosity | 3x10⁶ | 2x10⁶ | 2x10⁶ | 6x10⁵ |
| High shear viscosity | 30 | 20 | 10 | 3 |
| turbidity | 58.1 | 46.4 | 31.8 | 16.7 |

### Example 6: Different Second Polymers

All second polymers (bold type) were used at a level of 2.3% polymer solids.

**Table 8**

| | | | | | |
|---|---|---|---|---|---|
| Water | qs100 | qs100 | qs100 | qs100 | qs100 |
| TEA | 1.65 | 1.65 | 1.65 | 1.65 | 1.65 |
| **Second polymer #1** | **5.00** | - | - | - | - |
| **Second polymer #2** | - | **5.00** | - | - | - |
| **PVP/VA** | - | - | **4.6** | - | - |
| **PVP K90** | - | - | - | **2.3** | - |
| **Comp. 2^{nd} poly. #1** | - | - | - | - | **8.80** |
| Water | 15.00 | 25.00 | 15.00 | 15.00 | 15.00 |
| LAPONITE XLG | - | 0.50 | - | - | - |
| First poly. #1 | 6.00 | 6.00 | 6.00 | 6.00 | 6.00 |
| NEOLONE PE | 0.50 | 0.50 | 0.50 | 0.50 | 0.50 |
| Low shear stress viscosity | 6x10⁵ | 6x10⁵ | 2x10⁵ | 5x10⁵ | N/A |
| High shear stress viscosity | 3 | 15 | 2 | 6 | (phase separation) |
| turbidity | 16.7 | 30 | 327 | 37.7 | |

Second polymer #2 is a copolymer of 17% MAA, 28% MMA, 23% BA, 20% EA and 12% HEMA, with 0.6% DDM; 46% solids aqueous dispersion; Mw=90,000. Comp. 2^{nd} poly. #2 is a crosslinked MAA/MMA copolymer which does not contain any hydroxyalkyl (meth)acrylate.

**Table 9**

| | | | | |
|---|---|---|---|---|
| Water | qs100 | qs100 | qs100 | qs100 |
| TEA | 1.65 | 1.65 | 1.65 | 1.65 |
| **VP/MAM/VI** | **11.5** | - | - | - |
| **VP/DMAEM** | - | **11.5** | - | - |
| **VP/acrylates/LA** | - | - | **2.3** | - |
| **OAM/acrylates/BAEM** | - | - | - | **2.3** |
| Water | 15.00 | 25.00 | 15.00 | 15.00 |
| LAPONITE XLG | - | 0.50 | - | - |
| First poly. #1 | 6.00 | 6.00 | 6.00 | 6.00 |
| NEOLONE PE | 0.50 | 0.50 | 0.50 | 0.50 |
| Low shear stress viscosity | 15x10⁴ | 4x10⁵ | 6x10⁴ | 1x10⁶ |
| High shear stress viscosity | 1 | 10 | 100 | 10 |
| turbidity | 88.1 | 173.8 | 97.9 | 133.1 |

VP is vinylpyrrolidone, VI is vinylimidazole, DMAEM is dimethylaminoethyl methacrylate, BAEM is butylaminoethyl methacrylate,

### Comparative Example 3: Other Clays with First Polymer #1

**Table 10**

| | | | |
|---|---|---|---|
| Water | qs100 | qs100 | qs100 |
| TEA | 1.81 | 1.97 | 1.82 |
| Second polymer #1 | 5.00 | 5.00 | 5.00 |
| Water | 15.00 | 15.00 | 15.00 |
| BENTOLITE® WH | 0.50 | - | - |
| BENTONIT GEL WHITE® GP | - | 0.50 | - |
| BENTONIT GEL WHITE® H | - | - | 0.50 |
| First Polymer #1 | 6.00 | 6.00 | 6.00 |
| NEOLONE™PE | 0.50 | 0.50 | 0.50 |
| | | | |
| Low shear stress viscosity | 2x10⁵ | 1x10⁵ | 3x10⁵ |
| High Shear stress viscosity | 20 | 20 | 15 |
| turbidity | > 2000 | > 2000 | > 2000 |

**Table 11**

| | | | | |
|---|---|---|---|---|
| Water | qs100 | qs100 | qs100 | qs100 |
| TEA | 1.81 | 1.97 | 1.78 | 1.82 |
| Second polymer #1 | 5.00 | 5.00 | 5.00 | 5.00 |
| Water | 15.00 | 15.00 | 15.00 | 15.00 |
| OPTIGEL WX | 0.50 | - | - | - |
| OPTIGEL WM | - | 0.50 | - | - |
| MINERAL COLLOID MO | - | - | 0.50 | - |
| AEROSIL 300 | - | - | - | 0.50 |
| First Polymer #1 | 6.00 | 6.00 | 6.00 | 6.00 |
| NEOLONE™PE | 0.50 | 0.50 | 0.50 | 0.50 |
| | | | | |
| Low shear stress visco | 7x10⁵ | 1x10⁵ | 6x10⁵ | 6x10⁴ |
| High Shear stress visco | 20 | 25 | 30 | 20 |
| turbidity | 1332 | 1661 | > 2000 | 270 |

| | | | | |
|---|---|---|---|---|
| OPTIGEL WM has a particle size of ca. 1000 nm AEROSIL 300 has a surface area of 300 m²/g and a particle size of 7 nm. | | | | |

None of the clays listed in Tables 10 and 11 above provide simultaneously improvement of the rheology profile towards a more pseudo-plastic behavior with a low turbidity equivalent to what a consumer would describe as a transparent product.

### Example 7: Effect of Ethanol

**Table 12**

| | | |
|---|---|---|
| Water | qs100 | qs100 |
| TEA | 1.85 | 1.99 |
| Second polymer #1 | 5.0 | 5.0 |
| Water | 15.00 | 15.00 |
| LAPONITE® XLG | - | 0.50 |
| First polymer #1 | 6.00 | 6.00 |
| NEOLONE™PE | 0.50 | 0.50 |
| Ethanol | 10.00 | 10.00 |
| | | |
| Low shear stress viscosity | 3x10⁴ | 2x10⁶ |
| High Shear stress viscosity | 4 | 5 |
| turbidity | 7.9 | 26.5 |

## Claims

1. An aqueous composition having less than 1 wt% surfactant; said composition comprising:
(a) from 0.5-4 wt% of a first polymer comprising polymerized residues of: (i) from 5-30 wt% (meth)acrylate ester of a polyethylene glycol having 15 to 30 ethylene oxide residues and a C₁₀-C₂₂ alkyl group on one end; (ii) from 10-50% (meth)acrylic acid; and (iii) from 40-65 wt% C₁-C₈ alkyl (meth)acrylate;
(b) from 0.05-1.5 wt% of a synthetic clay having an average particle size from 20-90 nm and a surface area from 120-500 m²/g; and
(c) from 0.3-5 wt% of a second polymer comprising polymerized residues of: (i) from 5-35 wt% C₃-C₆ carboxylic acid monomer; (ii) from 5-20 wt% C₂-C₃ hydroxyalkyl (meth)acrylate; (iii) from 20-55 wt% C₁-C₄ alkyl methacrylate; and (iv) from 18-55 wt% C₂-C₄ alkyl acrylate;
wherein the aqueous composition has a pH from 5 to 10.

2. The composition of claim 1 in which the first polymer comprises polymerized residues of: (i) from 20-28 wt% (meth)acrylate ester of a polyethylene glycol having 17 to 23 ethylene oxide residues and a C₁₆-C₂₂ alkyl group on one end; (ii) from 20-27% (meth)acrylic acid; and (iii) from 46-55 wt% C₁-C₄ alkyl acrylate.

3. The composition of claim 2 comprising from 1-3 wt% of the first polymer.

4. The composition of claim 3 comprising from 0.5-3.5 wt% of the second polymer.

5. The composition of claim 4 in which the second polymer comprises polymerized residues of: (i) from 10-30 wt% C₃-C₆ carboxylic acid monomer; (ii) from 7-15 wt% C₂-C₃ hydroxyalkyl (meth)acrylate; (iii) from 25-51 wt% methyl or ethyl methacrylate; and (iv) from 20-47 wt% C₂-C₄ alkyl acrylate.

6. The composition of claim 5 comprising from 0.2-1 wt% of a synthetic clay.

7. The composition of claim 6 in which the synthetic clay has an average particle size from 20-30 nm.

8. The composition of claim 7 in which the synthetic clay has a surface area from 250-450 m²/g.

9. The composition of claim 8 containing from1.5-2.1 wt% of the first polymer.

10. The composition of claim 9 having a pH from 6 to 9.
